# EUROPEAN PATENT SPECIFICATION

(11) **EP 1 684 640 B1**
(45) Date of publication and mention of the grant of the patent: **28.09.2011**
(21) Application number: 04768890.8
(22) Date of filing: 15.10.2004
(51) Int. Cl.: A61B 17/128

(54) **APPLICATOR**
AUFBRINGVORRICHTUNG
APPLICATEUR

(30) Priority: 15.10.2003 GB 0324106; 15.07.2004 GB 0415796
(43) Date of publication of application: 02.08.2006
(73) Proprietor: FEMCARE LIMITED, Nottingham NG8 6AR (GB)
(72) Inventor: BRIGHT, Charles, Edward, Nr Burton-on-Trent Staffs. DE13 7EJ (GB)
(74) Representative: Baker, Colin John
(86) International application number: PCT/GB2004/004360
(87) International publication number: WO 2005/039422

(56) References cited:
- EP-A- 1 304 079
- US-A- 5 755 726
- US-B1- 6 610 073

## Description

The present invention relates to applicators and more particularly to applicators for insertion and closure of surgical clips. The document US-A-5 755 726 is regarded as the closest prior art.

Such clips may be for sterilisation or other medical, orthopaedic or surgical purposes where an intermediate position of an applicator or other appliance is required to be able to be accurately determined.

The term applicator is thus meant to include other appliances, particularly in the medical field, which involve a closure of a jaw or other similar gripping or squeezing action in which a specific intermediate position is required to be determined.

The present invention will now be described with reference to applicators for surgical clips. Except in open surgery it is necessary for the applicator to be inserted down a cannula for the more modem "key hole" surgery. Thus, the applicator or more precisely barrel portion of the applicator has to pass down the cannula loaded with a clip to be attached for occlusion of, for example a vas deferens or other tubular member.

With clips of the Filshie type such as disclosed in GB patent no. 2177748 excessive force applied to the clip during passage of the barrel down the cannula can result in clip failure. The clip could fall out of the barrel once it emerges from the cannula or alternatively the clip could fail by incorrect closure.

The present invention seeks to obviate this problem by providing a visual indication of an intermediate position of the applicator to ensure that there is no over-closure of a clip during passage of an applicator barrel down a cannula.

According to the present invention, there is provided an applicator according to claim 1.

The present invention therefore provides an applicator, said applicator comprising a barrel portion and a handle portion attached thereto, said barrel portion comprising means for holding a surgical clip and said handle comprising trigger means said trigger means being operative to effect closure of a surgical clip held within said barrel by movement, of said trigger from a first position to a second position at which second position said applicator is operative to effect closure of said clip, in which said trigger is provided with a visual indicator means indicative of an intermediate trigger position at which intermediate position said surgical clip held in said barrel is closed to a half closed position.

Preferably said visual indicator means comprises an indentation on said trigger.

Alternatively said visual indicator means comprises a reduced section of said trigger.

In a further embodiment said visual indicator means comprises groove means in said trigger.

In said further embodiment said groove means may comprise means for engaging with a removable stop means said removable stop means being co-operative with a portion of said handle to prevent movement of said trigger past a predetermined position.

Preferably said groove means is present on both sides of said trigger.

Preferably said stop means may comprise a U shaped member which is designed in a first locking position to sit within said groove means and to protrude from said groove means to form said stop means.

Preferably said U shaped member comprises spring steel or similar material ensuring secure engagement with said groove means.

Preferably said U shaped member comprises means for gripping by a surgeon to effect removal of said U shaped member from said trigger enabling movement of said trigger past said predetermined position.

In a further embodiment the trigger may comprise two or more colours arranged to identify specific portions of the trigger.

In a further embodiment the handle includes a recess the trigger being pivotally mounted within the handle the trigger being movable into said recess when said trigger is operated, in which the trigger is dimensioned to have minimal clearance between itself and the recess over substantially the whole pivotal movement of the trigger.

Preferably the visual indicator means is provided by a change of surface texture at a defined position across the trigger.

This change of surface texture will provide a visual indication of the defined position without any substantial variation of the surface dimension of the trigger.

The advantage design feature is that as the trigger is moved into and out of the handle there will be no appreciable gap between the trigger and the recess of the handle.

In surgical operation surgical gloves are worn and if there is any substantial gap between the trigger and the recess as the trigger is operated then there is a possibility of a glove catching between the trigger and the recess and being torn. This would create hygienic problems were it to occur and thus this feature is an important advantage of this embodiment.

The visual indication feature is further enhanced by the pressure felt by the surgeon on the trigger as the clip is partially closed.

Embodiments of the present invention will now be described by reference to the accompanying drawings in which:
Figure 1 shows an applicator for clips of the Filshie type;
Figure 2 shows in greater detail the operating handle of the applicator of Figure 1 in accordance with the present invention;
Figure 3 shows a first embodiment of the visual indicator means according to the present invention;
Figures 4a and 4b show a second embodiment with the trigger in two position;
Figure 5 shows a first embodiment of the U shaped member comprising the stop means;
Figure 6 shows a second embodiment of the U shaped member comprising said stop means,
Figure 7 shows a further embodiment of the application according to the present invention in perspective view,
Figure 7a and 7b showing a portion of the trigger and main body portion of the handle in cross section with an enlarged portion illustrating the change in texture feature,
Figure 8. shows a partial cross section of the applicator of Figure 7 illustrating the operation of the trigger and,
Figure 9 shows an exploded view of the applicator of Figure 7.

With reference now to the drawings an applicator 10 for applying clips of the Filshie type (as described in European patent application EP 1304079 A ) is shown. Such clips are of hinged type and will be hereinafter referred to as Filshie clips .

The applicator 10 comprises a barrel portion 12 and a handle portion 14 including a trigger 16.

The applicator is designed to pass down a relatively small diameter cannula 18.

In order to do so it is necessary, for the Filshie clip to pass down the cannula whilst in position in the applicator at the end of barrel 12.

A problem which arises is that if the clip is closed too far by application of trigger 16 then the clip may partially latch and the clip will not subsequently be able to.be correctly, attached.

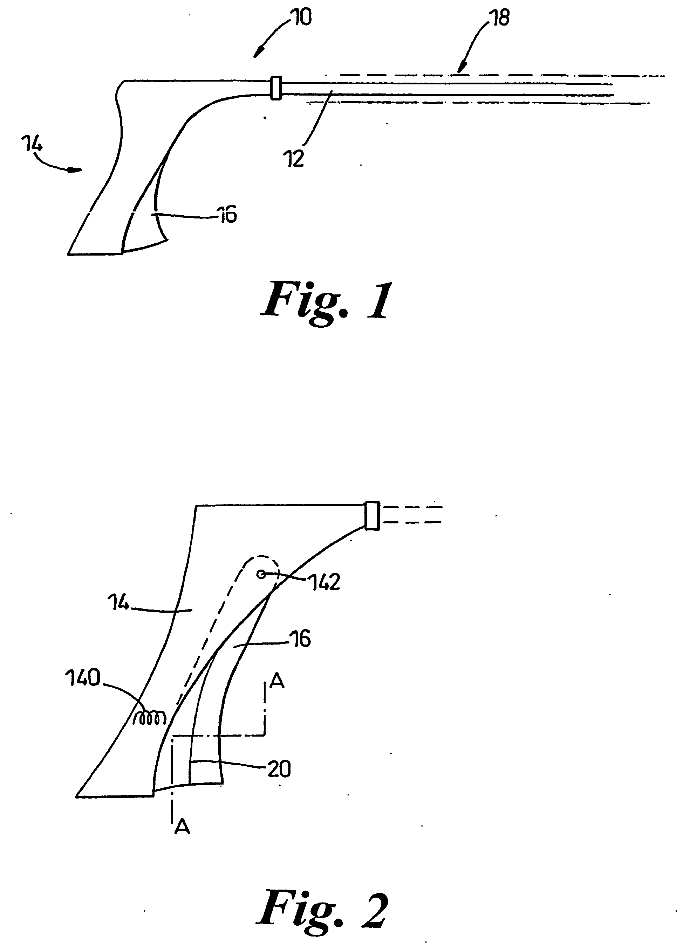

To enable the surgeon to ascertain the correct position of the trigger to achieve the half closed position the trigger and handle combination is provided with visual indication means 20 as indicated in Figure 2.

This visual indication means preferably comprises an elongate linear feature easily discernible by a surgeon enabling the surgeon to readily set the trigger to a correct position.

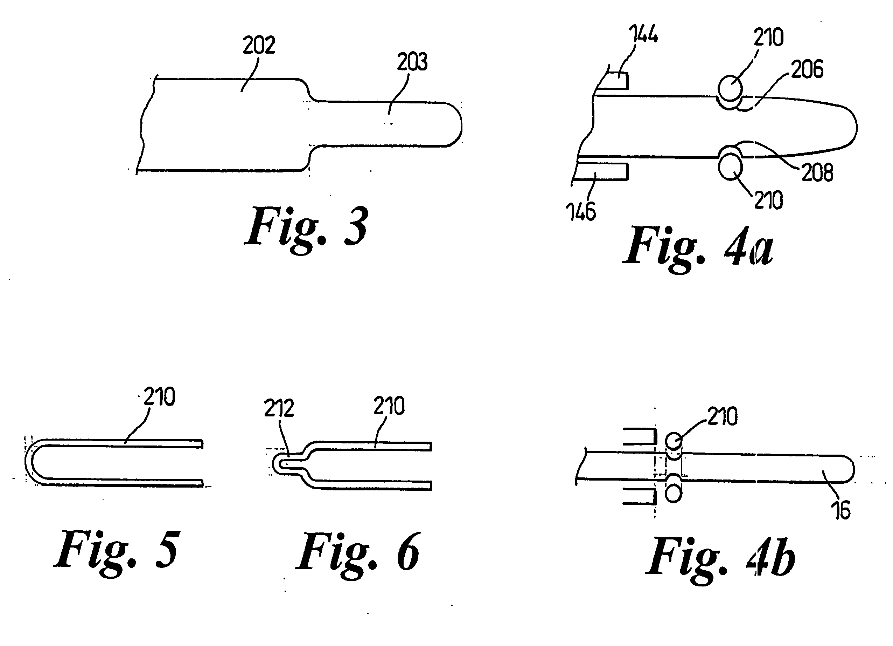

The visual indicator may also compose means for physically preventing further movement of the trigger past the correct semi-closed position of the clip this being illustrated hereinafter with reference to Figures 4, 5 and 6.

With reference to Figure 2 the visual indicator 20 marks a position at which with trigger 16 is moved on pivot 142 against for example return spring 140 the clip positioned at the distal end of barrel 12 will be in a semi-closed position.

Preferably the visual indicator line 20 will exist for a substantial distance over the trigger to provide a visual indication even when the trigger is being gripped by one or more of the digits of a surgeons hand.

In Figure 2 a section of the trigger along line A-A is shown for various embodiments illustrated in Figures 3 to 6.

In Figure 3 the trigger 14 is provided with a dual thickness shape shown at 202, 203 the change in thickness providing the visual indication 20.

In Figures 4a and 4b a groove 206, 208 is provided, preferably on both sides of the trigger.

The groove can provide the visual indication itself but in a specific embodiment a U shaped member 210 (see Figures 5 and 6) is inserted along the groove.

For illustrative purposes an edge portion 144, 146 of handle 14 is shown illustrating that when trigger 16 is pulled back the U shaped member 210 abuts the edges 144, 146 and physically prevents further movement of the trigger.

The U shaped member 210 is shown in a first embodiment in Figure 5 and preferably comprises a spring steel member allowing it to grip the grooves in trigger 16. Alternatively a suitable rigid plastic material may be used.

In Figure 6 the U shaped member is provided with gripping means 212 facilitating easy removal once capable closure of the Filshie clip is required.

The visual indication 20 may also be provided or enhanced by a change in colour of the trigger along line 20.

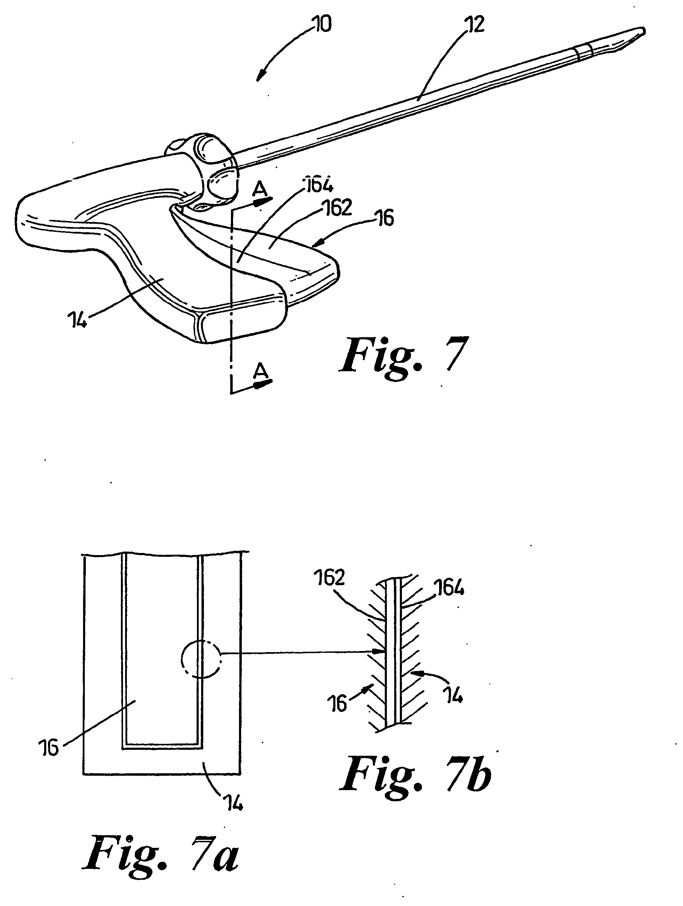

With reference now to Figure 7 in an alternative embodiment the trigger 16 comprises two separate surface features.

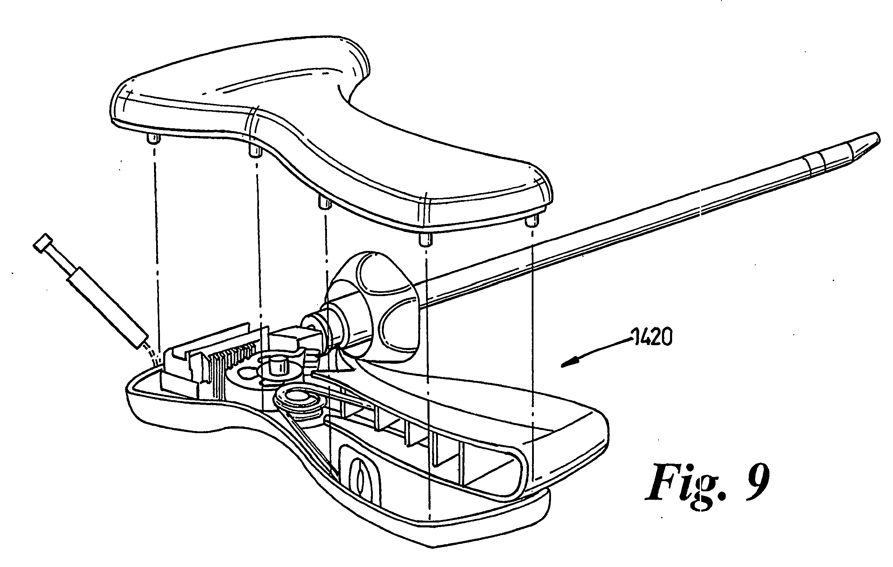

One part 162 (which could be the forward part on the rearward part) is provided with a surface texture whilst the other part 164 is a different surface texture which could for example be smooth. The advantage of this embodiment is that the trigger can be moulded from a single, preferably plastics, injection without use of a colouring agent in part of the trigger and also because the texturing can be made to raise or lower the surface by only a very small amount, possibly a few microns, the trigger can be made to slide into and out of the recess 1420 (shown exploded in Figure 9) with minimal clearance. This is shown clearly in Figure 7a and 7b, Figure 7a showing a cross section through a portion of the handle 14 and trigger 16 and Figure 7b showing a magnified view of a section of the trigger 16 showing the edges surfaces 162 and 164 and illustrating the minimal change in surface dimension.

This removes the possibility of snagging or catching the surgical gloves of a surgeon whilst operating the applicator.

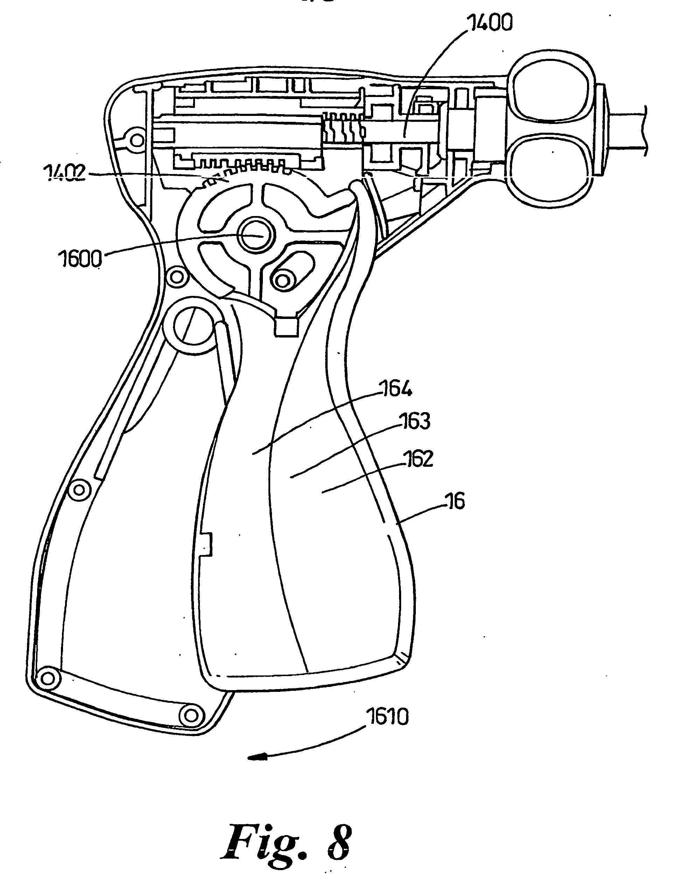

Additionally the amount of textured (or untextured) trigger left exposed as the trigger is pressed allows the surgeon a first appraisal of the level of clip closure and, as explained with reference to Figure 8 the applicator trigger will increasingly provide a feedback pressure as a clip is squeezed to a half closed position. Thus the surgeon can feel the clip pressure and look at the position of the textured line in the handle to provide visual and tactile approval of the position of the clip.

In Figure 8 the trigger 16 is movable on a pivot 1600 and drives the clip closure mechanism 1400 via a rack and pinion drive 1402.

As the trigger 16 moves in the direction of arrow 1610 to close the clip a feedback pressure is felt on the trigger 16 by the surgeon combining with the position of texture change line 163 to provide the surgeon with a tactile as well as visual indication.

Figure 9 shows the applicator in an exploded form to provide a further explanation of the embodiment of Figure 7.

## Claims

1. An applicator (10), said applicator (10) comprising a barrel portion (12) and a handle portion (14) attached thereto, said barrel portion (12) comprising means for holding a surgical clip and said handle (14) comprising trigger means (16) said trigger means (16) being operative to effect closure of a surgical clip held within said barrel (12) by movement of said trigger (16) from a first position to a second position at which second position said applicator (10) is operative to effect closure of said clip, said applicator (10) being further provided with a visual indicator means (20) indicative of an intermediate trigger position at which intermediate position said surgical clip held in said barrel (12) is closed to a half closed position, **characterised in that** said visual indicator means (20) are provided on said trigger (16).

2. An applicator as claimed in claim 1, in which said visual indicator means comprises an indentation on said trigger.

3. An applicator as claimed in claim 1, in which said visual indicator means comprises a reduced section of said trigger.

4. An applicator as claimed in claim 1, in which said visual indicator means comprises groove means in said trigger.

5. An applicator as claimed in claim 4, in which said groove means may comprise means for engaging with a removable stop means said removable stop means being co-operative with a portion of said handle to prevent movement of said trigger past a predetermined position.

6. An applicator as claimed in claim 5, in which said groove means is present on both sides of said trigger.

7. An applicator as claimed in claim 6, in which said stop means comprises a U shaped member which is designed in a first locking position to sit within said groove means and to protrude from said groove means to form said stop means.

8. An applicator as claimed in claim 7, in which said U shaped member comprises spring steel or similar material ensuring secure engagement with said groove means.

9. An applicator as claimed in claim 8, in which said U shaped member comprises means for gripping by a surgeon to effect removal of said U shaped member from said trigger enabling movement of said trigger past said predetermined position.

10. An applicator as claimed in one of claims 1 to 9, in which the trigger may comprise two or more colours arranged to identify a specific position of the trigger .

11. An applicator as claimed in Claim 1 in which the handle includes a recess the trigger being pivotally mounted within said handle to be moveable within said recess when said trigger is operated in which the trigger is dimensioned to have minimal clearance between itself and the recess edges over substantially the whole pivotal movement of the trigger.

12. An application is claimed in Claim 1 in which the visual indicator is provided by a change in surface texture at a defined position across the trigger.

13. An applicator as claimed in Claim 12 in which the trigger provides a feedback pressure as a clip is being moved from an open to a half closed position this feedback pressure providing a tactile sense to the surgeon which when combined with the visual indication provides additional information on the position of the clip.

14. An applicator as claimed in Claim 1, wherein said means for holding a surgical clip are gripping means at a distal end from said handle portion for gripping a surgical clip in which said handle portion includes trigger means operative to move into and out of a main body portion of said handle and being operative to move said gripping means to grip said clip

15. An applicator as claimed in any one of the preceding claims in which said visual indicator means comprises a change in surface texture at a defined intermediate position said surface texture change being dimensioned such that the clearance between the trigger means and said main body portion is minimal, thereby removing the possibility of snagging surgical gloves on movement of the trigger.

## Patentansprüche

1. Eine Aufbringvorrichtung (10), wobei die Aufbringvorrichtung (10) einen Rohrabschnitt (12) und einen Griffabschnitt (14), der daran angebracht ist, aufweist, wobei der Rohrabschnitt (14) Mittel zum Halten einer Operationsklemme aufweist und der Griff (14) Abzugsmittel (16) aufweist, wobei die Abzugsmittel (16) funktionsfähig sind, um ein Schließen einer in dem Rohr (12) gehaltenen Operationsklemme durch eine Bewegung des Abzugs (16) von einer ersten Position in eine zweite Position zu bewirken, an welcher zweiten Position die Aufbringvorrichtung (10) funktionsfähig ist, um ein Schließen der Klemme zu bewirken, wobei die Aufbringvorrichtung (10) ferner mit einem visuellen Anzeigemittel (20) versehen ist, das eine Zwischen-Abzugsposition anzeigt, an welcher Zwischenposition die in dem Rohr (12) gehaltene Operationsklemme in einer halb geschlossenen Position geschlossen ist, **dadurch gekennzeichnet, daß** die visuellen Anzeigemittel (20) an dem Abzug (16) vorgesehen sind.

2. Eine Aufbringvorrichtung gemäß Anspruch 1, bei der das visuelle Anzeigemittel eine Vertiefung an dem Abzug aufweist.

3. Eine Aufbringvorrichtung gemäß Anspruch 1, bei der das visuelle Anzeigemittel einen eingezogenen Abschnitt des Abzugs aufweist.

4. Eine Aufbringvorrichtung gemäß Anspruch 1, bei der das visuelle Anzeigemittel Nutmittel in dem Abzug aufweist.

5. Eine Aufbringvorrichtung gemäß Anspruch 4, bei der das Nutmittel Mittel zum Eingreifen mit einem entfernbaren Anschlagmittel aufweist, wobei das entfernbare Anschlagmittel mit einem Abschnitt des Griffs zusammenwirkt, um eine Bewegung des Abzugs über eine vorbestimmte Position hinaus zu verhindern.

6. Eine Aufbringvorrichtung gemäß Anspruch 5, bei der das Nutmittel an beiden Seiten des Abzugs vorhanden ist.

7. Eine Aufbringvorrichtung gemäß Anspruch 6, bei der das Anschlagmittel ein U-förmiges Element aufweist, das gestaltet ist, um in einer ersten Verriegelungsposition in dem Nutmittel zu sitzen und von dem Nutmittel zur Bildung des Anschlagmittels vorzustehen.

8. Eine Aufbringvorrichtung gemäß Anspruch 7, bei dem das U-förmige Element Federstahl oder ähnliches Material aufweist, das einen sicheren Eingriff mit dem Nutmittel sicherstellt.

9. Eine Aufbringvorrichtung gemäß Anspruch 8, bei der das U-förmige Element Mittel zum Greifen durch einen Chirurgen aufweist, um ein Entfernen des U-förmigen Elements von dem Abzug zu bewirken, um eine Bewegung des Abzugs über die vorbestimmte Position hinaus zu ermöglichen.

10. Eine Aufbringvorrichtung gemäß einem der Ansprüche 1 bis 9, bei dem der Abzug zwei oder mehr Farben aufweisen kann, die angeordnet sind, um eine bestimmte Position des Abzugs zu identifizieren.

11. Eine Aufbringvorrichtung gemäß Anspruch 1, bei der der Griff eine Ausnehmung aufweist und der Abzug schwenkbar in dem Griff angebracht ist, um in der Ausnehmung bewegbar zu sein, wenn der Abzug betätigt wird, wobei der Abzug so dimensioniert ist, daß er einen minimalen Abstand zwischen sich selbst und den Ausnehmungskanten über im wesentlichen der gesamten Schwenkbewegung des Abzugs besitzt.

12. Eine Aufbringvorrichtung gemäß Anspruch 11, bei dem der visuelle Anzeiger durch eine Änderung der Oberflächentextur an einer bestimmten Position über dem Abzug vorgesehen ist.

13. Eine Aufbringvorrichtung gemäß Anspruch 12, bei dem der Abzug einen Rückmeldungsdruck liefert, wenn eine Klemme von einer offenen in eine halb geschlossene Position bewegt wird, wobei dieser Rückmeldungsdruck eine taktile Wahrnehmung für den Chirurgen liefert, welche, in Kombination mit der visuellen Anzeige, eine zusätzliche Information über die Position der Klemme liefert.

14. Eine Aufbringvorrichtung gemäß Anspruch 1, wobei die Mittel zum Halten einer Operationsklemme Greifmittel an einem distalen Ende von dem Griffabschnitt zum Greifen einer Operationsklemme sind, wobei der Griffabschnitt Abzugsmittel aufweist, die funktionsfähig sind, um sich in einen Hauptkörperabschnitt des Griffes hinein und aus diesem heraus zu bewegen, und die funktionsfähig sind, um die Greifmittel zum Greifen der Klemme zu bewegen.

15. Eine Aufbringvorrichtung gemäß einem der vorstehenden Ansprüche, wobei das visuelle Anzeigemittel eine Änderung in der Oberflächentextur an einer definierten zwischenposition aufweist, wobei die Oberflächentexturänderung so dimensioniert ist, daß der Zwischenraum zwischen dem Abzugsmittel und dem Hauptkörperabschnitt minimal ist, wodurch die Möglichkeit eines Hängenbleibens von Operationshandschuhen bei der Bewegung des Abzugs beseitigt wird.

## Revendications

1. Applicateur (10), ledit applicateur (10) comprenant une portion de cylindre (12) et une portion de manche (14) attachée à celle-ci, ladite portion de cylindre (12) comprenant un moyen pour tenir une pince chirurgicale et ledit manche (14) comprenant un moyen déclencheur (16), ledit moyen déclencheur (16) étant fonctionnel pour effectuer une fermeture d'une pince chirurgicale tenue dans ledit cylindre (12), par un mouvement dudit déclencheur (16) depuis une première position jusqu'à une seconde position, à laquelle seconde position ledit applicateur (10) est fonctionnel pour effectuer une fermeture de ladite pince, ledit applicateur (10) étant en outre muni d'un moyen d'indication visuelle (20) indiquant une position de déclencheur intermédiaire, à laquelle position intermédiaire ladite pince chirurgicale tenue dans ledit cylindre (12) est fermée à une position à moitié fermée, **caractérisé en ce que** ledit moyen d'indication visuelle (20) est disposé sur ledit déclencheur (16).

2. Applicateur selon la revendication 1, dans lequel ledit moyen d'indication visuelle comprend une indentation sur ledit déclencheur.

3. Applicateur selon la revendication 1, dans lequel ledit moyen d'indication visuelle comprend une section réduite dudit déclencheur.

4. Applicateur selon la revendication 1, dans lequel ledit moyen d'indication visuelle comprend un moyen de gorge dans ledit déclencheur.

5. Applicateur selon la revendication 4, dans lequel ledit moyen de gorge peut comprendre un moyen permettant une mise en prise avec un moyen de butée amovible, ledit moyen de butée amovible coopérant avec une portion dudit manche pour empêcher un mouvement dudit déclencheur au-delà d'une position prédéterminée.

6. Applicateur selon la revendication 5, dans lequel ledit moyen de gorge est présent des deux côtés dudit déclencheur.

7. Applicateur selon la revendication 6, dans lequel ledit moyen de butée comprend un organe en forme de U qui est conçu dans une première position de blocage pour siéger dans ledit moyen de gorge et faire saillie à partir dudit moyen de gorge pour former ledit moyen de butée.

8. Applicateur selon la revendication 7, dans lequel ledit organe en forme de U comprend de l'acier à ressort ou un matériau similaire garantissant une mise en prise solide avec ledit moyen de gorge.

9. Applicateur selon la revendication 8, dans lequel ledit organe en forme de U comprend un moyen de préhension par un chirurgien pour effectuer le retrait dudit organe en forme de U dudit déclencheur, permettant un mouvement dudit déclencheur au-delà de ladite position prédéterminée.

10. Applicateur selon l'une des revendications 1 à 9, dans lequel le déclencheur peut comprendre deux couleurs ou plus, agencées pour identifier une position spécifique du déclencheur.

11. Applicateur selon la revendication 1, dans lequel le manche inclut un évidement, le déclencheur étant monté pivotant au sein dudit manche pour être mobile dans ledit évidement lorsque ledit déclencheur est actionné, dans lequel le déclencheur est dimensionné pour avoir un jeu minimal entre lui-même et les bords d'évidement sur quasiment l'intégralité du mouvement de pivotement du déclencheur.

12. Applicateur selon la revendication 11, dans lequel l'indication visuelle est formée par un changement de texture de surface à une position définie sur le déclencheur.

13. Applicateur selon la revendication 12, dans lequel le déclencheur fournit une pression de rétroaction à mesure qu'une pince est déplacée d'une position ouverte à une position à moitié fermée, cette pression de rétroaction donnant une sensation tactile au chirurgien qui, combinée à l'indication visuelle, fournit des informations additionnelles sur la position de la pince.

14. Applicateur selon la revendication 1, dans lequel ledit moyen pour tenir une pince chirurgicale est un moyen de préhension à une extrémité distale de ladite portion de manche pour empoigner une pince chirurgicale dans laquelle ladite portion de manche inclut un moyen déclencheur opérationnel pour se déplacer dans et hors d'une portion de corps principal dudit manche et opérationnel pour déplacer ledit moyen de préhension pour empoigner ladite pince.

15. Applicateur selon l'une quelconque des revendications précédentes, dans lequel ledit moyen d'indication visuelle comprend un changement de texture de surface en une position intermédiaire définie, ledit changement de texture de surface étant dimensionné de telle sorte que le jeu entre le moyen de déclencheur et ladite portion de corps principal soit minimal, supprimant ainsi la possibilité d'accrocher des gants chirurgicaux lors d'un mouvement du déclencheur.
